# EUROPÄISCHE PATENTSCHRIFT

(11) **EP 0 554 210 B1**
(45) Veröffentlichungstag und Bekanntmachung des Hinweises auf die Patenterteilung: **04.06.1997**
(21) Anmeldenummer: 93810005.4
(22) Anmeldetag: 06.01.1993
(51) Int. Cl.: A61F 2/34, A61B 17/58, A61B 17/16

(54) **Bausatz für eine künstliche Hüftgelenkspfanne**
Set of parts for an artificial hip acetabular cup
Jeu de pièces pour une coque acétabulaire artificielle de hanche

(30) Priorität: 29.01.1992 CH 249/92
(43) Veröffentlichungstag der Anmeldung: 04.08.1993
(73) Patentinhaber: Sulzer Orthopädie AG, 6340 Baar (CH)
(72) Erfinder: Schöller, Dietrich, Prof.Dr., W-5000 Köln (DE); Sandoz, Yvan, CH-8405 Winterthur (CH)
(74) Vertreter: Trieblnig, Adolf

(56) Entgegenhaltungen:
- EP-A- 0 147 339
- EP-A- 0 169 975
- EP-A- 0 313 762
- EP-A- 0 314 951
- EP-A- 0 341 198
- EP-A- 0 341 199
- EP-A- 0 353 171
- WO-A-85/02535
- WO-A-88/01491
- DE-U- 9 016 523
- US-A- 3 685 058

## Beschreibung

Die Erfindung betrifft einen Bausatz für eine künstliche Hüftgelenkspfanne, enthaltend eine metallische Aussenschale, eine in diese einsetzbare Innenschale und an der Aussenschale anbringbare Befestigungselemente, welche einen im Knochengewebe eines Beckenteils verankerbaren Sockel und eine im Knochengewebe befestigbare Schraubverankerung umfassen.

Ein aus der WO-A-85/02535 (Fig.7) bekannter Bausatz der genannten Art enthält einen Sockel in Form eines in das Knochengewebe einschraubbaren hohlzylindrischen Verankerungsteils, mit einem in diesen einschraubbaren Verbindungsteil, der einen entsprechend der Aussenfläche der Aussenschale gewölbten Stirnabschnitt aufweist, an dem die Aussenschale mittels einer ihre Wand durchsetzenden Schraube befestigt werden kann. Zusätzlich kann eine Schraubverankerung in Form einer Knochenschraube vorgesehen sein, die durch ein in der Wand der Aussenschale angeordnetes Schraubenloch in das Knochengewebe eingeschraubt werden kann. Durch die Befestigungselemente der bekannten Anordnung wird die Hüftgelenkspfanne in einer ihre Aussenschale eng umschliessenden, vorgängig in einen Hüftknochen eingearbeiteten kugelkalottenförmigen Vertiefung gehalten. Die bekannte Befestigungsanordnung setzt voraus, dass im Implantationsbereich eine für die Aufnahme der Aussenschale ausreichende, intakte Partie des Hüftknochens vorhanden ist. In Fällen, in denen diese Bedingung nicht gegeben ist, ist der bekannte Bausatz nicht verwendbar.

Bei einem aus der WO-A-88/01491 bekannten Bausatz ist die Aussenschale an zwei einander etwa gegenüberliegenden Stellen ihres Umfanges mit angeformten Ansätzen versehen, die je mit einer Bohrung zur Aufnahme eines in diese mit selbsthemmendem Festsitz einführbaren Zapfens ausgeführt sind, welcher von einem an einem Beckenteil befestigbaren plattenförmigen Anschlussteil absteht. Diese bekannte Ausführung ist jeweils als Ersatz für einen im Bereich des Hüftgelenks fehlenden Beckenteil vorgesehen, wobei die Aussenschale an ihrer dem Beckenteil zugekehrten Aussenfläche eine offenzellige Struktur zum Einwachsen von Knochenmaterial aufweist und die plattenartigen Halteteile im Schambein-Darmbeinbereich des Beckens angeschraubt werden sollen. Die mit angeformten Ansätzen versehene Aussenschale des bekannten Bausatzes ergibt einen relativ voluminösen Bauteil, dessen genaue Positionierung im Bereich des Hüftgelenks jeweils eine individuelle Anpassung der Form der Anschlussteile an die anatomischen Gegebenheiten und/oder eine relativ aufwendige Vorbereitung der entsprechenden, für die Aufnahme dieser plattenförmigen Anschlussteile relativ gross auszuführenden Partien des Beckens erfordert. Der bekannte Bausatz ist somit nur in beschränktem Masse an unterschiedliche anatomische Gegebenheiten im Implantationsbereich anpassbar, die, insbesondere hinsichtlich der Abmessungen des zu ersetzenden Beckenteils und der Winkelstellung der zu implantierenden Teile, in der Praxis innerhalb relativ weiter Grenzen variieren können. Um eine ständige Verfügbarkeit derartiger, in der Regel relativ selten gebrauchter Bausätze zu gewährleisten, ist daher eine relativ aufwendige Lagerhaltung eines Sortiments mit mehreren, in unterschiedlicher Grösse ausgeführten Teilen solcher Bausätze erforderlich, wobei im Bedarfsfall jeweils einer der Bausätze entsprechend den gegebenen anatomischen Verhältnissen ausgewählt, an diese Verhältnisse angepasst und schliesslich implantiert wird.

Der Erfindung liegt die Aufgabe zugrunde, einen insbesondere in dieser Hinsicht verbesserten Bausatz für eine Hüftgelenkspfanne in einer gegenüber bisherigen Ausführungen vereinfachten, möglichst kompakten Bauweise zu schaffen, welche die Verwendung einfach herstellbarer und auf einfache Weise verstellbarer Bauteile gestattet, die im wesentlichen ohne zusätzliche Bearbeitung an unterschiedliche anatomische Gegebenheiten angepasst und bezüglich des Bereichs des Hüftgelenks positioniert werden können.

Diese Aufgabe wird bei einem Bausatz der eingangs genannten Art erfindungsgemäss dadurch gelöst, dass die Befestigungselemente mindestens eine weitere Schraubverankerung umfassen, und dass der Sockel und die Schraubverankerungen je eine ausserhalb des Knochengewebes positionierbare Abstützung für die Aussenschale enthalten, wobei für jedes Befestigungselement unterschiedlich grosse Abstützlängen zwischen Aussenschale und Knochengewebe wählbar sind.

Die erfindungsgemässe Ausführung ergibt einen Bausatz in einer kompakten Bauweise, welche durch die im wesentlichen radial zur Aussenschale einstellbaren Befestigungselemente einen verbesserten, direkten Kraftfluss zwischen der Hüftgelenkspfanne und den die Befestigungselemente aufnehmenden Bereichen des Beckens gewährleistet. Dabei ist durch relativ einfach auszuführende Bohrungen im Knochengewebe eine sichere Verankerung der Befestigungselemente des erfindungsgemässen Bausatzes erzielbar, durch welche je eine Komponente der Stützkraft - unter entsprechend geringer Beanspruchung des Knochengewebes - in die betreffende Beckenpartie eingeleitet wird. Die mit unterschiedlichen Abstützlängen ausführbaren Befestigungselemente gestatten auf einfache Weise eine optimale, während der Implantation gegebenenfalls korrigierbare Einstellung der Hüftgelenkspfanne.

Ausgestaltungen des Erfindungsgegenstandes sind in den abhängigen Patentansprüchen angegeben.

Weitere Merkmale und Einzelheiten ergeben sich aus der folgenden Beschreibung von in der Zeichnung schematisch dargestellten Ausführungsbeispielen der Erfindung, in Verbindung mit den Patentansprüchen. In der Zeichnung zeigen:
- Fig. 1: Teile eines erfindungsgemäss ausgebildeten Bausatzes in einem bezüglich der Aussenschale diametral verlaufenden Schnitt;
- Fig. 2: weitere Teile des Bausatzes nach Fig. 1 in einem gegenüber der Darstellung nach Fig. 1 in Umfangsrichtung der Aussenschale versetzten, radialen Teilschnitt;
- Fig. 3: eine Einzelheit eines Bausatzes gemäss Fig. 1 in einer abgewandelten Ausführungsform, in einer der Fig. 2 entsprechenden Darstellung;
- Fig. 4: einen Teil eines Bausatzes gemäss Fig. 1 in einer abgewandelten Ausführungsform, in einer Draufsicht;
- Fig. 5: den Teil nach Fig. 4 in einer Seitenansicht;
- Fig. 6: Teile des Bausatzes nach Fig. 1 mit einer Hilfsvorrichtung zum Vorbereiten von zur Aufnahme der anderen Teile des Bausatzes bestimmten Partien des Knochengewebes.

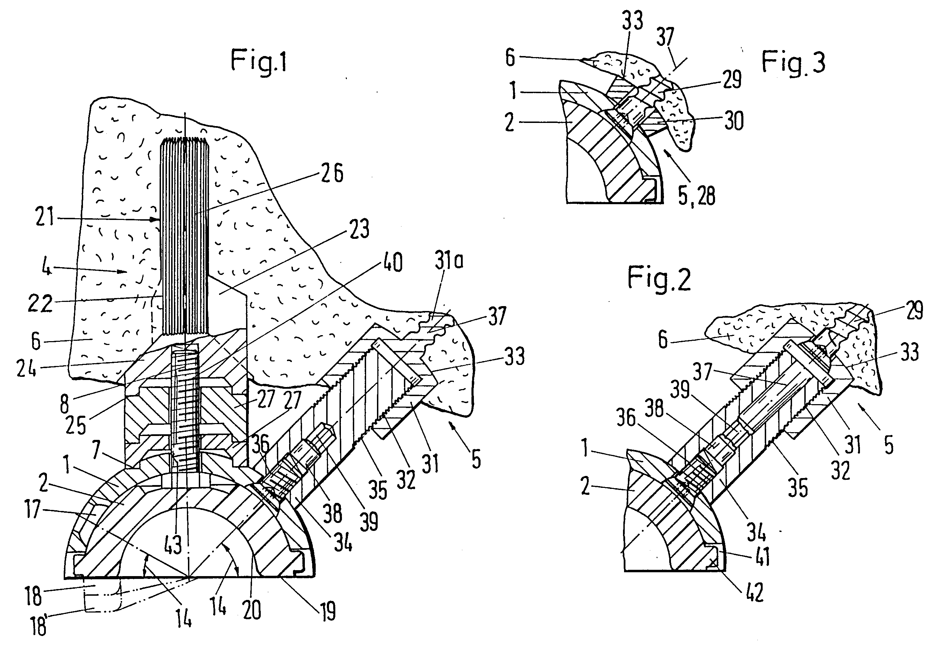

Der Bausatz nach den Figuren 1 und 2 enthält eine metallische Aussenschale 1 in Form einer hohlen Halbkugel, eine in diese einsetzbare, ebenfalls halbkugelförmig ausgeführte Innenschale 2 und an der Aussenschale 1 radial abstehend anbringbare Befestigungselemente in Form eines Sockels 4 und zweier Schraubverankerungen 5, welche im Knochengewebe 6 eines menschlichen Beckenteils befestigbar sind und durch welche die Aussenschale 1 ausserhalb des Knochengewebes 6 positionierbar ist. Der Bausatz ist als Ersatz für einen fehlenden Beckenteil im Bereich des Hüftgelenks vorgesehen.

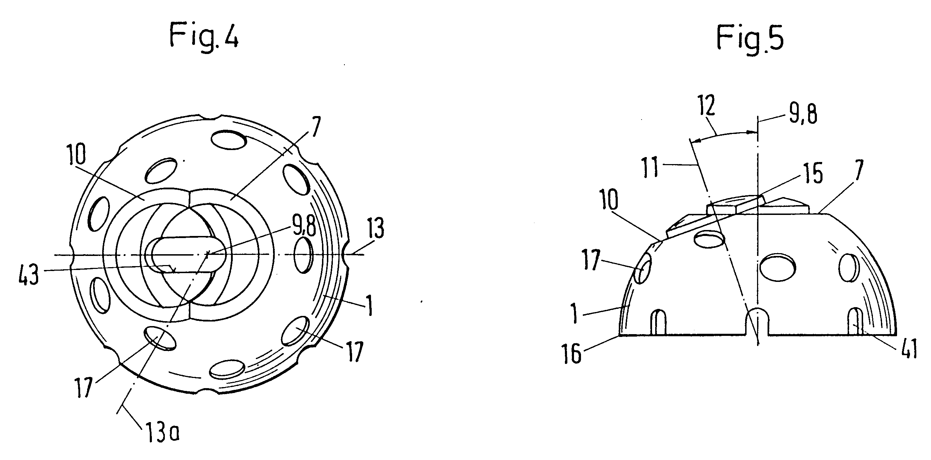

Die Aussenschale 1 ist mit einer im Bereich ihres Pols 15 vorgesehenen Bohrung 43 zur Aufnahme einer Befestigungsschraube 40 für den Sockel 4 sowie mit einer zur Bohrung 43 konzentrischen, kreisringförmigen Basisfläche 7 zum Abstützen des Sockels 4 ausgeführt, welche senkrecht zu einer Zentrierachse 8 angeordnet ist, die darstellungsgemäss mit der Polachse 9 der Aussenschale 1 zusammenfällt. Zur Aufnahme des Sockels 4 in einer anderen, gegenüber der Polachse 9 radial versetzten Winkelstellung kann die Aussenschale 1 mit mindestens einer gegenüber der Basisfläche 7 geneigten weiteren Basisfläche 10 versehen sein, welche sich entsprechend der Darstellung nach den Figuren 4 und 5 mit der Basisfläche 7 überschneidet und welche senkrecht zu einer zweiten Zentrierachse 11 angeordnet ist, die mit der Zentrierachse 8 bzw. der Polachse 9 einen Winkel 12 einschliesst und mit dieser darstellungsgemäss in einer Symmetrieebene 13 der Aussenschale 1 liegen kann. Wie aus der Fig. 4 hervorgeht, ist bei dieser Ausführung die Bohrung 43 als Langloch ausgebildet, welches entsprechende, je zu einer der Basisflächen 7 oder 10 konzentrische Einstellungen der Befestigungsschraube 40 gestattet. Es können auch weitere, entsprechend geneigte Basisflächen 10 vorgesehen sein. Innerhalb der durch die Abmessungen der Aussenschale 1 und des Sockels 4 konstruktiv gegebenen Grenzen ist der Winkel 12 frei wählbar. Die dargestellte Ausführung, bei der der Winkel 12 bis zu 20° betragen kann, gestattet in den meisten vorkommenden Fällen eine optimale Anpassung an die jeweils gegebenen anatomischen Verhältnisse.

Die Aussenschale 1 ist zwischen ihrem Pol 15 und ihrem Äquator 16 ferner mit mehreren, in Umfangsrichtung gegeneinander versetzt angeordneten, radialen Durchgangslöchern 17 versehen, die mit zur Ebene des Äquators 16 in unterschiedlichen Winkeln 14 geneigten Achsen 37 ausgeführt sind. Die Durchgangslöcher 17 sind je zur Aufnahme einer Befestigungsschraube 36 für eine der auf der Aussenschale 1 in entsprechend unterschiedlichen Positionen anbringbaren Schraubverankerungen 5 (Fig. 1, 2) oder zur Aufnahme einer unmittelbar im Beckenbereich verankerbaren Knochenschraube 29 (Fig. 3) bestimmt. Dabei ist es zweckmässig, wenn die Durchgangslöcher 17, wie in Fig. 4 dargestellt, bezüglich der Symmetrieebene 13 symmetrisch auf der Aussenschale 1 angeordnet sind, so dass diese sowohl für den rechten als auch für den linken Hüftgelenksbereich verwendbar ist.

Die Aussenschale 1 kann im Bereich des Äquators 16 mit mehreren, darstellungsgemäss acht, über den Umfang verteilten Ausnehmungen 41 ausgeführt sein, die zur Aufnahme von an der Innenschale 2 vorgesehenen Nocken 42 bestimmt sind. Die Innenschale 2, die ebenfalls metallisch oder, wie in der Zeichnung dargestellt, aus einem Kunststoff ausgeführt sein kann, ist über die Nocken 42 in unterschiedlichen Winkelstellungen um die Polachse 9 mit der Aussenschale 1 verklinkbar. Dabei kann die Innenschale 2 mit einer in der Fig. 1 strichpunktiert angedeuteten einseitigen Ueberhöhung 18 bzw. 18' ausgeführt sein, welche um ein vorbestimmtes Mass über den Rand 19 der Aussenschale 1 vorsteht und durch welche dementsprechend auch bei ungünstigen anatomischen Verhältnissen im Hüftgelenksbereich, die z.B. die Positionierung der Hüftgelenkspfanne nur in einer funktionsmässig ungünstigen Winkelstellung gestatten, eine ausreichend sichere Führung der von der inneren Halbkugelfläche 20 aufzunehmenden Gelenkkugel gewährleistet werden kann.

Der Sockel 4 enthält einen im Knochengewebe 6 befestigbaren zylindrischen oder konischen Verankerungsteil 21 mit einem nach aussen konisch sich verbreiternden Kragenteil 24, zwei zylindrische Distanzstücke 27 und die Befestigungsschraube 40, durch welche die Aussenschale 1 über die Distanzstücke 27 gegen dem Kragenteil 24 verspannbar ist. Der Verankerungsteil 21 ist mit einer strukturierten Oberfläche 22 ausgeführt, die darstellungsgemäss aus kleinen, entsprechend den Mantellinien des zylindrischen Verankerungsteils 21 in dessen Längsrichtung, parallel zur Zentrierachse 8, verlaufenden Schneiden 26 gebildet sein kann. Der Verankerungsteil 21 und der Kragenteil 24 sind ferner über mindestens eine radial gestellte Längsflosse 23, darstellungsgemäss zwei entsprechende Längsflossen 23, miteinander verbunden, welche je in einen im Knochengewebe 6 anzubringenden Schlitz einführbar sind, so dass der Verankerungsteil 21 gegen Verdrehen gesichert gehalten ist. Der Kragenteil 24 und die Distanzstücke 27 sind je mit einer zur Basisfläche 7 bzw. 10 der Aussenschale 1 komplementären Kragenfläche 25 ausgeführt, wobei die Distanzstücke 27 je mit einer der Basisfläche 7 bzw. 10 entsprechenden Stützfläche versehen sind. Zweckmässig ist eine Lagerhaltung mehrer derartiger Distanzstücke 27 mit unterschiedlichen Dicken, welche bei entsprechender Kombination eine relativ feine Abstufung der Stützlänge des Sockels 4 und damit eine genaue Positionierung der Aussenschale 1 in Richtung der Zentrierachse 8 gestatten.

Die Schraubverankerung 5 nach Fig. 1, die an einem beliebigen Durchgangsloch 17, darstellungsgemäss in der Symmetrieebene 13, der Aussenschale 1 angebracht sein kann, enthält eine in das Knochengewebe 6 einschraubbare Hülse 31 mit einem Innengewinde 32, in welcher ein Zwischenstück 34 mit einem entsprechenden Aussengewinde 35 in Richtung der Achse 37 des betreffenden Durchgangslochs 17 kontinuierlich verstellbar gehalten ist. Das Zwischenstück 34 ist mit der Aussenschale 1 über eine lösbare Verschraubung verbunden, welche eine von der Innenseite der Aussenschale 1 her in das Zwischenstück 34 einschraubbare Befestigungsschraube 36 enthält, deren Schraubenkopf darstellungsgemäss mit einem Innensechskant ausgeführt sein kann. Der Innenhohlraum 38 ist mit Kontaktflächen 39, darstellungsgemäss in Form eines Innensechskants, zur Aufnahme eines durch das Durchgangsloch 17 einführbaren Steckschlüssels ausgeführt, durch den - bei gelöster Befestigungsschraube 36 - das Zwischenstück 34 in einem beliebigen Drehwinkel bezüglich der Achse 37 einstellbar ist, wodurch eine entsprechende Tiefenverstellung des Zwischenstücks 34 in der Hülse 31 erzielbar ist. Die Hülse 31 kann gemäss Fig. 1 mit einer von ihrer Stirnseite 33 abstehenden, z.B. angeformten, Knochenschraube 31a ausgeführt sein, durch welche die Stirnseite 33 gegen eine entsprechende Stützfläche einer die Hülse 31 aufnehmenden Ausnehmung des Knochengewebes angepresst werden kann.

Die zweite Schraubverankerung 5 des Bausatzes nach Fig. 1, welche auf der Aussenschale 1 gegenüber der vorstehend beschriebenen ersten Schraubverankerung 5 in Umfangsrichtung versetzt, z.B. in einer in Fig. 4 strichpunktiert angedeuteten Radialebene 13a, angebracht ist, kann in der vorstehend beschriebenen Weise oder entsprechend der Darstellung nach Fig. 2 ausgeführt sein. Bei der Schraubverankerung 5 nach Fig. 2, die sich nur geringfügig von der entsprechenden Ausführung nach Fig. 1 unterscheidet, ist das Zwischenstück 34 mit einer an den Innenraum 38 anschliessenden axialen Bohrung versehen, wodurch das Gewicht des Zwischenstücks 34 entsprechend verringert wird, und die Hülse 31 ist mit einer stirnseitigen Bohrung zur Aufnahme einer losen, in das Knochengewebe 6 einschraubbaren Knochenschraube 29 versehen, durch welche die Hülse 31 mit ihrer Stirnfläche 33 an das Knochengewebe 6 angepresst wird. Es versteht sich, dass auch anstelle der in Fig. 1 dargestellten ersten Schraubverankerung 5 eine entsprechende Schraubverankerung 5 gemäss Fig. 2 verwendet werden kann.

Entsprechend den jeweils gegebenen anatomischen Verhältnissen, insbesondere bei relativ geringem Abstand zwischen der Aussenschale 1 und dem benachbarten Beckenbereich, kann mindestens eine der beiden Schraubverankerungen 5 durch eine in Fig. 3 dargestellte einfache Verschraubung 28 gebildet sein, welche mindestens eine zwischen dem Knochengewebe 6 und der Aussenschale 1 anbringbare Distanzscheibe 30 passender Dicke und eine Knochenschraube 29 enthält, die von der Innenseite der Aussenschale 1 her durch das betreffende Durchgangsloch 17 und eine entsprechende Bohrung der Distanzscheibe 30 in das Knochengewebe 6 eingeschraubt werden kann.

Zweckmässig ist es, wenn dem Chirurgen ein Vorrat aus mehreren entsprechenden, mit unterschiedlichen Dicken ausgeführten Distanzscheiben 30 und mehreren, gegebenenfalls mit unterschiedlichen Längen ausgeführten Zwischenstücken 34 zur Verfügung steht, so dass bei relativ geringem Lageraufwand ein rascher Zugriff zu den den jeweils vorliegenden anatomischen Verhältnissen entsprechend auszuwählenden oder gegebenenfalls miteinander zu kombinierenden Teilen gewährleistet werden kann.

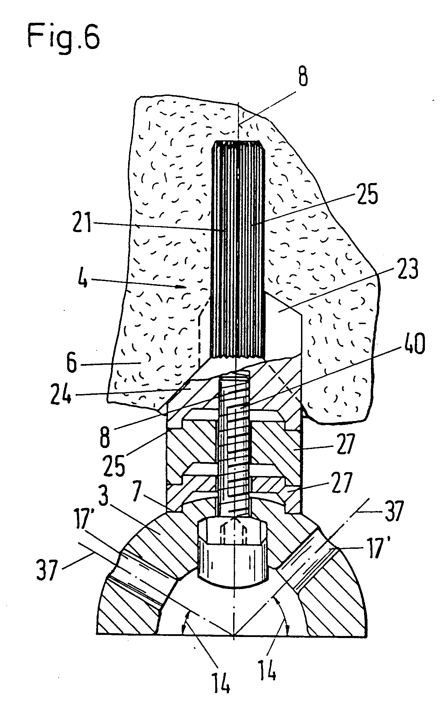

Zur Vorbereitung der Implantation der künstlichen Hüftgelenkspfanne werden im vorbestimmten Beckenbereich dem Kern des Sockels 4 entsprechende Ausnehmungen - eine dem Verankerungsteil 21 und dem Kragenteil 24 entsprechende Bohrung sowie den Längsflossen 23 entsprechende Schlitze - in das Knochengewebe 6 gearbeitet. In diese Ausnehmungen wird hierauf der Sockel 4 eingeschlagen. Entsprechend der Darstellung nach Fig. 6 wird in einem nächsten Schritt über passende Distanzstücke 27 mittels der Befestigungsschraube 40 eine Bohrlehre 3 auf dem Sockel 4 befestigt. Die Bohrlehre 3 entspricht in ihren Anschlussmassen der in einem späteren Verfahrensschritt in ihrer endgültigen Einbaulage anzubringenden Aussenschale 1.

Die Bohrlehre 3 ist in ebenfalls in Form einer hohlen Halbkugel sowie mit Durchgangslöchern 17' zur Führung jeweils eines Bohrwerkzeugs ausgeführt, deren Anordnung derjenigen der Durchgangslöcher 17 in der zu implantierenden Aussenschale 1 entspricht. Wie aus der Fig. 6 weiter hervorgeht, kann die Bohrlehre 3 mit einer wesentlich grösseren Wandstärke ausgeführt sein als die Aussenschale 1. Entsprechend wird durch die Durchgangslöcher 17' jeweils eine sichere, winkelgetreue Führung des Bohrwerkzeugs gewährleistet. Für die Anbringung der als zusätzliche Abstützung vorgesehenen Schraubverankerungen 5 kann die um die Zentrierachse 8 drehbare Bohrlehre 3 in einer Position fixiert werden, in der zwei passende Durchgangslöcher 17' gegen die zur Aufnahme der Schraubverankerungen 5 vorgesehenen Beckenpartien orientiert sind. Durch das in diese Durchgangslöcher 17' einführbare Bohrwerkzeug werden hierauf jeweils die Bohrungen für die Knochenschrauben 29 bzw. 31a und stirnseitige Anpressflächen für die Stirnseite 33 der Distanzscheiben 30 und/oder der Hülsen 31 im Knochengewebe 6 angebracht.

Bei ungünstigen anatomischen Verhältnissen, welche, z.B. wegen Fehlens einer geeigneten Knochenpartie, die Anbringung einer zweiten Schraubverankerung nicht gestatten, kann auch durch eine einzige Schraubverankerung 5, in Verbindung mit dem Sockel 4, eine den Umständen entsprechend ausreichende Fixierung der Hüftgelenkspfanne gewährleistet werden. Es versteht sich, dass gegebenenfalls auch eine dritte Schraubverankerung 5 vorgesehen sein kann.

Nach Anbringung der Bohrungen im Knochengewebe 6 kann die Bohrlehre 3 vom Sockel 4 entfernt werden, worauf an den Anpressflächen je eine Hülse 31 der verstellbaren Schraubverankerung mittels einer Knochenschraube 29 bzw. 31a im Knochengewebe 6 fixiert und in die Hülsen 31 je eines der Zwischenstücke 34 eingeschraubt werden kann. Wenn es die anatomischen Verhältnisse gestatten, kann anstelle mindestens einer der verstellbaren Schraubverankerungen 5 eine Einfachverschraubung 28 gemäss Fig. 3 verwendet werden, welche keine Hülse 31 enthält. In einem weiteren Verfahrensschritt wird die Aussenschale 1 in ihrer definitiven Endlage mit dem Sockel 4 über die Distanzstücke 27 verbunden, wobei bei Verwendung einer Einfachverschraubung 28 zwischen die Anpressfläche des Knochengewebes 6 und die Aussenschale 11 eine passende Distanzscheibe 30 eingelegt wird, über welche die Aussenschale 1 mittels der Knochenschraube 29 gegen das Knochengewebe 6 verspannt wird. In einem weiteren Verfahrensschritt wird hierauf bei der bzw. jeder kontinuierlich verstellbaren Schraubverankerung 5 das Zwischenstück 34 mit einem Steckschlüssel soweit aus der betreffenden Hülse 31 herausgeschraubt, bis es bündig an der Aussenschale 1 anliegt, und anschliessend mit der Aussenschale 1 starr verschraubt wird. Schliesslich wird die Innenschale 2 in die Aussenschale 1 eingesetzt und über die in die Ausnehmungen 41 einrastenden Nocken 42 verklinkt. Bei Innenschalen 2 mit einseitig überhöhtem Rand 19 ist dabei auf die passende Winkellage der Ueberhöhung 18, 18' zu achten.

## Patentansprüche

1. Bausatz für eine künstliche Hüftgelenkspfanne, enthaltend eine metallische Aussenschale (1), eine in diese einsetzbare Innenschale (2) und an der Aussenschale (1) anbringbare Befestigungselemente, welche einen im Knochengewebe (6) eines Beckenteils feststehend verankerbaren Sockel (4) und eine im Knochengewebe (6) befestigbare Schraubverankerung (5) umfassen,
dadurch gekennzeichnet, dass die Befestigungselemente mindestens eine weitere Schraubverankerung (5) umfassen, und dass der Sockel (4) und die Schraubverankerungen (5) je eine ausserhalb des Knochengewebes (6) positionierbare Abstützung für die Aussenschale (1) enthalten, wobei für jedes Befestigungselement unterschiedlich grosse Abstützlängen zwischen Aussenschale (1) und Knochengewebe (6) wählbar sind.

2. Bausatz nach Anspruch 1, mit einer an der Aussenschale (1) ausgebildeten Basisfläche (7) für die Aufnahme des Sockels (4),
dadurch gekennzeichnet, dass die Basisfläche (7) senkrecht zu einer Zentrierachse (8) angeordnet ist, welche mit der Polachse (9) der Aussenschale (1) zusammenfällt.

3. Bausatz nach Anspruch 2, dadurch gekennzeichnet, dass für die Aufnahme des Sockels (4) mindestens eine weitere Basisfläche (10, Fig. 4) auf der Aussenschale (1) angebracht ist, deren Zentrierachse (11) in einem Winkel (12) von bis zu 20° von der Polachse (9) wegsteht und mit dieser in einer Symmetrieebene (13) der Aussenschale liegt.

4. Bausatz nach einem der Ansprüche 1 bis 3, dadurch gekennzeichnet, dass die Aussenschale (1) rundherum auf unterschiedlichen Winkeln (14) zwischen Aequator (16) und Pol (15) Durchgangslöcher (17) aufweist, um die Schraubverankerungen (5) in verschiedenen Positionen anbringen zu können.

5. Bausatz nach Anspruche 4, dadurch gekennzeichnet, dass die Durchgangslöcher (17) bezüglich der Symmetrieebene (13) symmetrisch auf der Aussenschale (1) verteilt sind.

6. Bausatz nach einem der Ansprüche 1 bis 5, dadurch gekennzeichnet, dass die Innenschale (2) bei Drehung um die Polachse (9) in unterschiedlichen Drehwinkeln mit der Aussenschale (1) verklinkbar ist.

7. Bausatz nach Anspruch 6, dadurch gekennzeichnet, dass die Innenschale (2) einseitig eine Ueberhöhung (18) des Randes (19) über eine innere Halbkugelfläche (20) hinaus aufweist.

8. Bausatz nach einem der Ansprüche 2 bis 7, dadurch gekennzeichnet, dass der Sockel (4) im Knochengewebe (6) einen zylindrischen oder konischen Verankerungsteil (21) mit strukturierter Oberfläche (22), mit mindestens einer ausgeprägten Längsflosse (23) und mit einem verbreiterten Kragenteil (24) aufweist, welcher in eine zur Basisfläche (7, 10) komplementäre Kragenfläche (25) übergeht, wobei der Verankerungsteil (21) in einem beliebigen Drehwinkel um die Zentrierachse (8, 11) der Basisfläche (7, 10) auf der Aussenschale (1) befestigbar ist.

9. Bausatz nach Anspruch 8, dadurch gekennzeichnet, dass die strukturierte Oberfläche (22) aus kleinen, zur Mantelfläche des zylindrischen oder konischen Verankerungsteils (21) längs orientierten Schneiden (26) besteht, die sich beim Einschlagen des Verankerungsteils (21) in das Knochengewebe (6) eingraben.

10. Bausatz nach Anspruch 8, dadurch gekennzeichnet, dass als Abstützung für die Aussenschale (1) zwischen diese und den Kragenteil (24) einbaubare Distanzstücke (27) mit zur Basisfläche (7, 10) und zur Kragenfläche (25) passenden Gegenflächen vorgesehen sind.

11. Bausatz nach einem der Ansprüche 1 bis 10, bei dem eine der Schraubverankerungen (5) eine durch ein Durchgangsloch (17) der Aussenschale (1) in das Knochengewebe (6) einschraubbare Knochenschraube (29) enthält, dadurch gekennzeichnet, dass als Abstützung für die Aussenschale (1) mindestens eine zwischen diese und das Knochengewebe (6) einbaubare und in ihrer Dicke auswählbare Distanzscheibe (30) vorgesehen ist.

12. Bausatz nach einem der Ansprüche 1 bis 10, dadurch gekennzeichnet, dass eine der Schraubverankerungen (5) eine in das Knochengewebe (6) einschraubbare Hülse (31) mit einer an das Knochengewebe (6) anpressbaren Stirnseite (33) und einem Innengewinde (32) enthält, und dass als Abstützung für die Aussenschale (1) ein in der Hülse (31) kontinuierlich verstellbares Zwischenstück (34) mit einem dazu passenden Aussengewinde (35) vorgesehen ist, wobei das Zwischenstück (34) über eine lösbare Verschraubung (36) in einem beliebigen Drehwinkel bezüglich der Achse (37) eines Durchgangsloches (17) von der Innenseite der Aussenschale (1) fixierbar ist.

13. Bausatz nach Anspruch 12, dadurch gekennzeichnet, dass das Zwischenstück (34) einen Innenhohlraum (38) mit Kontaktflächen (39) für einen durch das Durchgangsloch (17) einführbaren Steckschlüssel aufweist, durch den bei gelöster Verschraubung (36) das Zwischenstück (34) in der Hülse (31) verstellbar ist.

## Claims

1. A set of parts for an artificial acetabulum containing a metallic outer shell (1), an inner shell (2) which can be inserted therein and attachment elements which can be mounted on the outer shell (1), and which comprise a support (4) which can be securely anchored in the osseous tissue (6) of a pelvic part and a screw attachment (5) which can be fixed in the osseous tissue (6),
**characterised in that** the attachment elements comprise at least one further screw attachment (5),
**and in that** the support (4) and the screw attachments (5) each contain a support for the outer shell (1) which can be positioned outside the osseous tissue, with it being possible to chose support lengths of different dimensions between outer shell (1) and osseous tissue (6) for each attachment element.

2. A set of parts according to Claim 1, having a base (7) for receiving the support (4) and constructed on the outer shell (1),
**characterised in that** the base (7) is disposed perpendicular to a centring axis (8), which coincides with the polar axis (9) of the outer shell (1).

3. A set of parts according to Claim 2,
**characterised in that** for receiving the support (4) at least one further base (10, Fig. 4) is mounted on the outer shell (1), the centring axis (11) of which is removed by an angle (12) of up to 20° from the polar axis (9) and lies therewith in a plane of symmetry (13) on the outer shell.

4. A set of parts according to one of Claims 1 to 3,
**characterised in that** the outer shell (1) comprises through-holes (17) all around at different angles between equator (16) and pole (15), so that it is possible to mount the screw attachments (5) in various positions.

5. A set of parts according to Claim 4,
**characterised in that** the through-holes (17) are distributed symmetrically over the outer shell (1) with respect to the plane of symmetry (13).

6. A set of parts according to one of Claims 1 to 5,
**characterised in that** the inner shell (2) can be interlocked with the outer shell (1) when rotated around the polar axis (9) in various angles of rotation.

7. A set of parts according to Claim 6,
**characterised in that** on one side the inner shell (2) comprises a raised part (18) of the edge (19) beyond an inner hemispherical surface (20).

8. A set of parts according to one of Claims 2 to 7,
**characterised in that** the support (4) in the osseous tissue (6) comprises a cylindrical or conical attachment part (21) having a structured surface (22), having at least one prominent longitudinal fin (23) and having a widened collar part (24), which passes into a collar surface (25) complementary to the base (7, 10), with it being possible to fix the attachment part (21) on the outer shell (1) in any angle of rotation around the centring axis (8, 11) of the base (7, 10).

9. A set of parts according to Claim 8,
**characterised in that** the structured surface (22) consists of small cutters (26), which are longitudinally orientated with respect to the generated surface of the cylindrical or conical attachment part (21) and which become embedded in the osseous tissue (6) when the attachment part (21) is forced in.

10. A set of parts according to Claim 8,
**characterised in that** spacers (27) having counter-surfaces fitting the base (7, 10) and the collar surface (25) are provided as a support for the outer shell (1) between the latter and the collar part (24).

11. A set of parts according to one of Claims 1 to 10 in which one of the screw attachments (5) contains a bone screw (29), which can be screwed into the osseous tissue (6) through a through-hole (17) on the outer shell (1),
**characterised in that** as a support for the outer shell (1) at least one spacer disc (30) is provided, which can be installed between said shell and the osseous tissue (6) and whose thickness can be chosen.

12. A set of parts according to one of Claims 1 to 10,
**characterised in that** one of the screw connections (5) contains a sleeve (31), which can be screwed into the osseous tissue (6), having a front end (33) which can be pressed against the osseous tissue (6) and an internal thread (32),
**and in that** as a support for the outer shell (1) an adaptor (34) continuously adjustable in the sleeve (31) having a matching external thread (35) is provided, with it being possible to fix the adaptor (34) from the inside of the outer shell (1) via a detachable screw connection (36) in any angle of rotation with respect to the axis (37) of a through-hole (17).

13. A set of parts according to Claim 12,
**characterised in that** the adaptor (34) comprises an internal cavity (38) having contact surfaces (39) for a socket spanner which can be inserted through the through-hole (17), by which the adaptor (34) can be adjusted in the sleeve (31) when the screw connection (36) is unscrewed.

## Revendications

1. Jeu de pièces pour une coque acétabulaire artificielle de hanche, contenant une cuvette extérieure (1) métallique, une cuvette intérieure (2) susceptible de s'insérer dans celle-ci et des éléments de fixation susceptibles d'être montés sur la cuvette extérieure (1) et comprenant un socle (4), susceptible d'être ancré à demeure dans le tissu osseux (6) d'une partie bassin, et un ancrage à vissage (5) susceptible d'être fixé dans le tissu osseux, caractérisé en ce que les éléments de fixation comprennent au moins un autre ancrage à vissage (5) et en ce que le socle (4) et les ancrages à vissage (5) contiennent chacun un appui, susceptible d'être positionné hors du tissu osseux (6), pour la cuvette extérieure (1), des longueurs d'appui différentes étant susceptibles d'être choisies pour chaque élément de fixation, entre cuvette extérieure (1) et tissu osseux (6).

2. Jeu de pièces selon la revendication 1, avec une surface de base (7) réalisée sur la cuvette extérieure (1), pour recevoir le socle (4), caractérisé en ce que la surface de base (7) est disposée perpendiculairement par rapport à un axe de centrage (8) qui coïncide avec l'axe polaire (9) de la cuvette extérieure (1).

3. Jeu de pièces selon la revendication 1, caractérisé en ce que, pour recevoir le socle (4), est ménagée sur la cuvette extérieure (1) au moins une surface de base supplémentaire (10, figure 4)), dont l'axe de centrage (11) s'écarte sous un angle (12) allant jusqu'à 20° vis-à-vis de l'axe polaire (9) et est situé avec celui-ci dans un plan de symétrie (13) de la cuvette extérieure.

4. Jeu de pièces selon l'une des revendications 1 à 3, caractérisé en ce que la cuvette extérieure (1) présente tout autour, à des angles (14) différents, des trous de passage (17) entre l'équateur (16) et le pôle (15), pour pouvoir monter les ancrages à vissage (5) dans différentes positions.

5. Jeu de pièces selon la revendication 4, caractérisé en ce que les trous de passage (17) sont répartis symétriquement sur la cuvette extérieure (1), par rapport au plan de symétrie (13).

6. Jeu de pièces selon l'une des revendications 1 à 5, caractérisé en ce que la cuvette intérieure (2) peut être encliquetée en différentes positions angulaires avec la cuvette extérieure (1), lors d'une rotation autour de l'axe polaire (9).

7. Jeu de pièces selon la revendication 6, caractérisé en ce que la cuvette intérieure (2) présente d'un côté un bombage (18) de la bordure (19), au-dessus d'une surface interne (20) hémisphérique.

8. Jeu de pièces selon l'une des revendications 2 à 7, caractérisé en ce que le socle (4) présente dans le tissu osseux (6) une partie d'ancrage (21) cylindrique ou conique, ayant une surface (22) structurée, avec au moins un élément plan longitudinal (23) en relief, et avec une partie collerette (24) élargie qui se transforme en une surface de collerette (25) complémentaire de la surface de base (7, 10), la partie d'ancrage (21) étant susceptible d'être fixée sous un angle de rotation quelconque autour de l'axe de centrage (8, 11) de la surface de base (7, 10) sur la cuvette extérieure (1).

9. Jeu de pièces selon la revendication 8, caractérisé en ce que la surface structurée (22) est constituée de petits tranchants (26) orientés longitudinalement par rapport à la surface d'enveloppe de la partie d'ancrage (21) cylindrique ou conique et s'enfouissant dans le tissu osseux (6) lors de l'introduction par frappe de la partie d'ancrage (21).

10. Jeu de pièces selon la revendication 8, caractérisé en ce que, à titre d'appui pour la cuvette extérieure (1), sont prévues entre celle-ci et la partie collerette (24) des pièces d'écartement (27) susceptibles d'être insérées et ayant des surfaces conjuguées s'adaptant à la surface de base (7, 10) et à la surface de collerette (25).

11. Jeu de pièces selon l'une des revendications 1 à 10, pour lequel l'un des ancrages à vissage (5) contient une vis à os (29) susceptible d'être vissée dans le tissu osseux (6) en passant par un trou de passage (17) de la cuvette extérieure (1), caractérisé en ce qu'est prévu, comme appui pour la cuvette extérieure (1), au moins un disque d'écartement (30) susceptible d'être monté entre celle-ci et le tissu osseux (6) et dont l'épaisseur peut être sélectionnée.

12. Jeu de pièces selon l'une des revendications 1 à 10, caractérisé en ce que l'un des ancrages à vissage (5) comporte une douille (31) susceptible d'être vissée dans le tissu osseux (6), avec une face frontale (33) susceptible d'être pressée contre le tissu osseux (6) et avec un taraudage (32), et en ce que, à titre d'appui, pour la cuvette extérieure (1), est prévue une pièce intermédiaire (34), réglage de façon continue dans la douille (31) et avec un filetage mâle (35) s'y adaptant, la pièce intermédiaire (34) étant susceptible d'être fixée par l'intermédiaire d'un vissage (36) désolidarisable, selon un angle de rotation quelconque par rapport à l'axe (37) d'un trou de passage (17), ceci depuis le côté intérieur de la cuvette extérieure (1).

13. Jeu de pièces selon la revendication 12, caractérisé en ce que la pièce intermédiaire (34) comporte un espace creux intérieur (38) avec des surfaces de contact (39) pour une clé à canon multiforme, susceptible d'être introduite à travers le trou de passage (17) et au moyen de laquelle la pièce intermédiaire (34) est réglable dans la douille (31) lorsque le vissage (36) est désolidarisé.
